# EUROPEAN PATENT SPECIFICATION

(11) **EP 4 021 881 B1**
(45) Date of publication and mention of the grant of the patent: **10.05.2023**
(21) Application number: 20761908.1
(22) Date of filing: 20.08.2020
(51) Int. Cl.: C07C 51/215, C07C 51/43, C07C 63/26

(54) **APPARATUS FOR PURIFYING TEREPHTHALIC ACID**
VORRICHTUNG ZUR AUFREINIGUNG VON TEREPHTHALSÄURE
APPAREIL DE PURIFICATION D'ACIDE TÉRÉPHTALIQUE

(30) Priority: 28.08.2019 US 201962892548 P; 22.11.2019 US 201962939001 P
(43) Date of publication of application: 06.07.2022
(73) Proprietor: Koch Technology Solutions UK Limited, 4th Floor London EC2V 7JE (GB)
(72) Inventor: WARD, Phil, Wilmington, Delaware 19808 (US); ZHENG, Donghui, Wilmington, Delaware 19808 (US)
(74) Representative: Jones, Nicholas Andrew
(86) International application number: PCT/IB2020/057820
(87) International publication number: WO 2021/038394

(56) References cited:
- WO-A1-93/24440
- US-A- 5 840 965

## Description

### RELATED APPLICATIONS

This application claims priority to US Provisional Application No. 62/892548 filed on August 28, 2019 and 62/939001 filed on November 22, 2019,

### FIELD

This invention relates to an improved process for purifying terephthalic acid.

### BACKGROUND

In the production of purified terephthalic acid (PTA) by paraxylene oxidation, crude terephthalic acid (CTA) is initially formed in an acetic acid containing solvent media and then transferred into an aqueous media for purification. This transfer from an acetic acid to an aqueous media can be done by filtration and drying or by solvent interchange employing counter-current washing. Devices suitable for this solvent interchange can be filters employing either gas or liquid as the motive fluid for filtration.

U.S. Patent No. 5,840,965 to Turner et al., discloses a process for production of purified terephthalic acid, wherein an aqueous solution of crude terephthalic acid is subjected to hydrogenation to reduce impurities, crystallized to produce a slurry of purified terephthalic acid in an aqueous liquor, and carrying out an integrated separation washing process.

Usually the CTA employed in the production process of PTA is derived from the oxidation of paraxylene in a liquid reaction medium containing acetic acid to produce a slurry of CTA in the reaction medium. The liquid reaction medium normally incorporates a catalyst, for example a cobalt/manganese/bromide catalyst system which is soluble in the reaction medium. Suitably the oxidation is carried out in the presence of an oxygen source for example air, at a pressure of 5 to 30 bars absolute (bara), and preferably an oxygen concentration of 0 to 8% by volume in the gas leaving the reactor and at a temperature of 150 to 250°C. It is suitably a continuous process, and is preferably carried out in a stirred reactor. The reaction is exothermic and the heat of the reaction may conveniently be removed by evaporation of water and acetic acid from the reaction medium.

Following the oxidation step, the CTA produced may then be separated from the reaction medium conventionally by centrifugal separation or filtration followed by drying and then CTA reslurry in an aqueous medium for purification. This CTA transfer step, from the reaction medium to an aqueous medium, is typically referred to as solvent interchange. However, it is more expedient to exchange the reaction medium, preferably continuously, for an aqueous medium in a pressure filter. Rotary pressure filters utilizing both hydraulic (liquid) or pneumatic (gas) pressure as the motive force for filtration have been utilized in commercial PTA production processes for this duty, with the pneumatic (gas) application being of interest here.

Traditionally, high pressure oxidation reactor off-gas is available on PTA plants for general inerting duties, product conveying and pressure control etc., and it is this gas that is readily available for use in as a pneumatic fluid in filtration. However, to make this off-gas widely suitable for reuse in the PTA process, the reactor off-gas is traditionally treated for environmental compliance and dried, which has a number of disadvantages with respect to its use in the solvent interchange filtration process. For example, as noted above, the off-gas has typically undergone catalytic treatment in order to meet emissions regulations, particularly where used for PTA powder conveying, which is unnecessary for this duty due to separate treatment required for gases discharged to atmosphere from low pressure process systems. Treating the gas twice adds capital costs for unnecessary capacity. Also, gas pressure is typically low (below 4 bara) after treatment, limiting flexibility in the filtration solvent interchange equipment. Likewise, the treated gas is also dried in order to make it suitable for other duties and will therefore lead to unwanted evaporative cooling, leading to precipitation and fouling due to solvent being saturated with organics, if used for filtration without a water re-wetting.

Thus, it would be convenient to use untreated oxidation reactor off-gas directly as a source of motive pressure for counter-current solvent exchange for CTA in a terephthalic acid purification process, if a suitable and inexpensive process for conditioning the off-gas can be developed.

### SUMMARY

Provided is an apparatus for the purification of crude terephthalic acid, including a paraxylene oxidation reactor in which paraxylene is reacted with air in an acetic acid-based solvent containing catalyst to form a slurry containing crude terephthalic acid (CTA) crystals and an oxygen-depleted air off-gas, one or more crystallization vessels downstream of the reactor for flash cooling the slurry by pressure letdown, an off-gas conditioning system for removing and conditioning a portion of the oxygen-depleted air off-gas exiting the reactor, a rotary pressure filter for effecting solvent interchange in a first filtration step with counter-current washing utilizing the removed and conditioned oxygen-depleted air off-gas to facilitate CTA exchange from reaction liquor to an aqueous liquor, thereby creating an aqueous CTA slurry, dissolution of the CTA in the aqueous exchange solvent by heating to produce a CTA-containing aqueous solution, and a CTA purification reactor in which the CTA-containing aqueous solution is contacted with hydrogen under reducing conditions and at elevated temperature and pressure to reduce chemically at least some impurities present in the CTA to form purified terephthalic acid (PTA).

In one form, the off-gas conditioning system comprises an inlet pipe connected downstream of the condensers and feeding an off-gas conditioning vessel, said off-gas conditioning vessel having an outlet pipe connected to an inlet of the rotary pressure filter.

In another form, the off-gas conditioning system further comprises a flow control valve and a pressure control valve along the inlet pipe to feed the off-gas to the off-gas conditioning vessel.

In another form, the off-gas conditioning system further comprises a temperature control valve connected between an off-gas heater which is downstream of the condensers and the outlet pipe from the off-gas conditioning vessel.

In yet another form, the flow control valve, the pressure control valve and the temperature control valve are controlled by a flow control loop, to condition and maintain a desired dew point of the off-gas in the off-gas conditioning vessel.

### BRIEF DESCRIPTION OF THE DRAWINGS

The present disclosure is susceptible to various modifications and alternative forms, specific exemplary implementations thereof have been shown in the drawings and are herein described in detail. It should be understood, however, that the description herein of specific exemplary implementations is not intended to limit the disclosure to the particular forms disclosed herein.

This disclosure is to cover all modifications and equivalents as defined by the appended claims. It should also be understood that the drawings are not necessarily to scale, emphasis instead being placed upon clearly illustrating principles of exemplary embodiments of the present invention. Moreover, certain dimensions may be exaggerated to help visually convey such principles. Further where considered appropriate, reference numerals may be repeated among the drawings to indicate corresponding or analogous elements. Moreover, two or more blocks or elements depicted as distinct or separate in the drawings may be combined into a single functional block or element. Similarly, a single block or element illustrated in the drawings may be implemented as multiple steps or by multiple elements in cooperation.

The forms disclosed herein are illustrated by way of example, and not by way of limitation, in the figures of the accompanying drawings and in which like reference numerals refer to similar elements and in which:
FIG. 1 presents an overall flow diagram of a CTA production process utilizing continuous solvent interchange by filtration and without CTA drying; and
FIG. 2 presents a detailed flow diagram for part of the reactor off-gas treatment system and an off-gas conditioning system as shown simplistically in FIG. 1.

### DETAILED DESCRIPTION

Various aspects will now be described with reference to specific forms selected for purposes of illustration. It will be appreciated that the spirit and scope of the apparatus, system and methods disclosed herein are not limited to the selected forms. Moreover, it is to be noted that the figures provided herein are not drawn to any particular proportion or scale, and that many variations can be made to the illustrated forms.

Each of the following terms written in singular grammatical form: "a," "an," and "the," as used herein, may also refer to, and encompass, a plurality of the stated entity or object, unless otherwise specifically defined or stated herein, or, unless the context clearly dictates otherwise. For example, the phrases "a device," "an assembly," "a mechanism," "a component," and "an element," as used herein, may also refer to, and encompass, a plurality of devices, a plurality of assemblies, a plurality of mechanisms, a plurality of components, and a plurality of elements, respectively.

Each of the following terms: "includes," "including," "has," "'having," "comprises," and "comprising," and, their linguistic or grammatical variants, derivatives, and/or conjugates, as used herein, means "including, but not limited to."

Throughout the illustrative description, the examples, and the appended claims, a numerical value of a parameter, feature, object, or dimension, may be stated or described in terms of a numerical range format. It is to be fully understood that the stated numerical range format is provided for illustrating implementation of the forms disclosed herein, and is not to be understood or construed as inflexibly limiting the scope of the forms disclosed herein.

Moreover, for stating or describing a numerical range, the phrase "in a range of between about a first numerical value and about a second numerical value," is considered equivalent to, and means the same as, the phrase "in a range of from about a first numerical value to about a second numerical value," and, thus, the two equivalently meaning phrases may be used interchangeably.

Concentrations, dimensions, amounts, and other numerical data may be presented herein in a range format. It is to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited. For example, a range of about 1 to about 200 should be interpreted to include not only the explicitly recited limits of 1 and about 200, but also to include individual sizes such as 2, 3, 4, etc. and sub-ranges such as 10 to 50, 20 to 100, etc. Similarly, it should be understood that when numerical ranges are provided, such ranges are to be construed as providing literal support for claim limitations that only recite the lower value of the range as well as claims limitation that only recite the upper value of the range. For example, a disclosed numerical range of 10 to 100 provides literal support for a claim reciting "greater than 10" (with no upper bounds) and a claim reciting "less than 100" (with no lower bounds). In the figures, like numerals denote like, or similar, structures and/or features; and each of the illustrated structures and/or features may not be discussed in detail herein with reference to the figures. Similarly, each structure and/or feature may not be explicitly labeled in the figures; and any structure and/or feature that is discussed herein with reference to the figures may be utilized with any other structure and/or feature without departing from the scope of the present disclosure.

It is also to be understood that all technical and scientific words, terms, and/or phrases, used herein throughout the present disclosure have either the identical or similar meaning as commonly understood by one of ordinary skill in the art, unless otherwise specifically defined or stated herein. Phraseology, terminology, and, notation, employed herein throughout the present disclosure are for the purpose of description and should not be regarded as limiting.

CTA purification typically comprises adding an aqueous medium to the CTA to form a slurry thereof which is then heated to dissolve the CTA in the medium to provide an aqueous solution of terephthalic acid. This solution is then passed to a reduction step in which the solution is contacted with hydrogen under reducing conditions in the presence of a heterogeneous catalyst to reduce chemically organic impurities, for example 4-carboxybenzaldehyde (4-CBA). The hydrogenated solution is passed to pressure let-down vessels in which PTA crystals form to provide a slurry of PTA in aqueous medium. PTA is recovered from the aqueous medium.

The presently disclosed process includes oxidizing paraxylene with air in an acetic acid-based solvent containing catalyst in a reactor to form a slurry containing crude terephthalic acid (CTA) crystals and an oxygen-depleted air off-gas, and subsequently flash cooling the slurry by pressure letdown. A portion of the oxygen-depleted air exiting the reactor is removed and conditioned to provide the motive force for a filtration-based solvent interchange. Subsequently, the solvent interchange is effected in a first filtration step with counter-current washing utilizing the removed oxygen-depleted air in a rotary pressure filter, followed by CTA slurry heating to produce a CTA-containing solution which is contacted with hydrogen under reducing conditions and at elevated temperature and pressure to reduce chemically at least some of the impurities present in the CTA. Afterwards, the process includes reducing pressure and temperature to obtain a slurry comprising crystallized purified terephthalic acid (PTA) in aqueous medium, recovering the PTA in a second filtration step to produce a wet PTA cake, and heating the PTA cake in a rotary steam tube drier to produce a drier PTA product.

The oxygen-depleted air must be removed from the paraxylene oxidation reaction together with sufficient liquid to ensure constant wetting of the oxygen-depleted air and pressure let down equipment (PCV) to ensure the water saturated oxygen-depleted air does not become dry. This is necessary because untreated reactor off-gas contains brominated species that can hydrolyze to form highly corrosive hydrobromic acid in humid conditions. Accordingly, after conditioning it is preferable to control the temperature of the oxygen-depleted air above its dew point in order to prevent any remaining hydrogen bromide being concentrated up by evaporation.

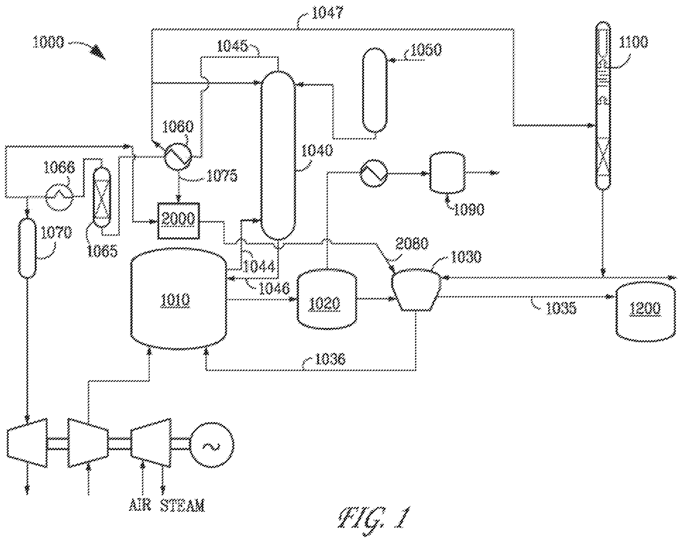

Referring now to FIG. 1, usually the CTA employed in the production process 1000 is derived from the oxidation of paraxylene in a liquid reaction medium containing acetic acid in reactor 1010, to produce a slurry of CTA in the reaction medium. The liquid reaction medium normally incorporates a catalyst, for example a cobalt/manganese/bromide catalyst system which is soluble in the reaction medium. Suitably the oxidation is carried out in the presence of an oxygen source for example air, at a pressure of about 5 to about 30 bars absolute (bara), such as from about 8 to about 13 bara, and preferably an oxygen concentration of from 0 to about 8% by volume in the gas leaving the reactor, and at a temperature of 150 to 250° C, such as from about 170 to about 200° C. It is suitably a continuous process, and is preferably carried out in a stirred reactor. The reaction is exothermic and the heat of the reaction may conveniently be removed by evaporation of water and acetic acid from the reaction medium.

The water and acetic acid evaporated from the reaction medium leave the reactor 1010 through line 1044 and are preferably sent to a rectification column 1040 to produce acetic acid having a lower water content. Acetic acid having a lower water content obtained in this way may be returned to the oxidation step through line 1046 and water and oxygen-depleted air off-gas recovered from rectification are sent through line 1045, and passed through a condenser train 1060. The water recovered in condenser train 1060 is sent through line 1047 to water recovery column 1100. The recovered water may be used as the aqueous medium for solvent exchange and dissolving CTA and/or as the aqueous wash liquor for washing PTA. Rectification column 1040 also has an inlet for aqueous liquor returning from the CTA purification section entering from treatment vessel 1050.

Following the oxidation step, the CTA slurry is then flash cooled by pressure letdown in crystallization vessels 1020. Vaporized solvent from vessel 1020 is passed through a condenser and into a solvent treatment vessel 1090 prior to reuse. Subsequently, the CTA slurry is sent to one or more rotary pressure filters 1030, where the acidic reaction medium is solvent exchanged, preferably continuously, for an aqueous medium to provide a terephthalic acid stream comprising CTA in aqueous medium in the rotary pressure filter 1030. The CTA/aqueous medium leaves rotary pressure filter 1030 through line 1035 and passes to the terephthalic acid purification section 1200. The exchanged solvent leaves rotary pressure filter 1030 and is recycled to oxidation reactor 1010 via line 1036.

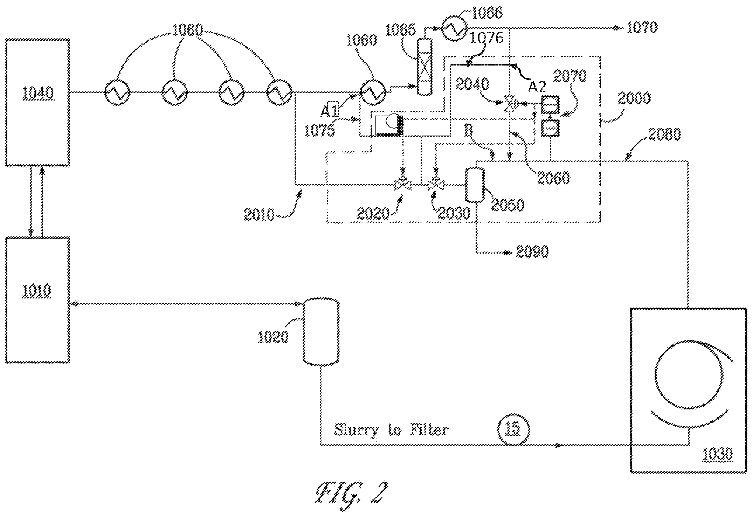

The oxygen-depleted air off-gas recovered from condenser train 1060 is fully saturated with water and a low level of assorted organic compounds, and is sent to off-gas treatment system 1065, 1066 and then 1070, where residual organics and carbon monoxide are removed prior to discharge to atmosphere or for use as treated off-gas within the PTA process. Now referring also to FIG 2, which shows the gas conditioning system 2000 in more detail, a portion of the saturated oxygen-depleted air off-gas is removed from the off-gas at point A1, after at least one of the condensers in condenser train 1060, or at point up to point A2 after off-gas heater 1066. Careful selection of the appropriate removal point and ensuring this gas remains fully wetted with condensate across pressure let down valve 2030 is necessary to ensure appropriate control of gas corrosivity and adequate gas cooling (to around 90 to 110°C). Removal at point A1 via line 1075 or at point A2 via line 1076, and re-wetting with an excess of condensate taken via overheads condensate line 2010 is preferred. This ensures an appropriate temperature in the off-gas conditioning vessel 2050 together with an appropriately minimized level of corrosive species in the gas. After optimizing the off-gas for temperature, pressure and dew point, the conditioned off-gas is sent through line 2080 to the one or more rotary pressure filters 1030 to serve as the motive force for solvent exchange and filtration.

Turning now to detail in FIG. 2, the off-gas condition system 2000 comprises a number of lines to effect temperature, pressure and dew point modification of the off-gas, such that it is suitable as a driving gas for the rotary pressure filters 1030. The conditioning step is specified to ensure an adequately wet gas to minimize evaporative cooling but also to prevent 100% saturation in order to eliminate the potential for fog formation in the filtration equipment. This condition is achieved by reducing overheads untreated off-gas pressure to the pressure required for supply to filtration across pressure control valve 2030 along with sufficient overheads condensate flow 2010, regulated by flow control valve 2020, to ensure a saturated gas. This saturated gas is then passed through a conditioning vessel 2050 before mixing with sufficient heated gas 2060 taken from the start of the off-gas treatment train after passing through absorber 1065 and off-gas heater 1066, regulated by temperature control valve 2040, to maintain an appropriate temperature margin above the dew point (or fogging) temperature. Efficient operation of this system to achieve appropriately conditioned gas is controlled by a flow control loop 2070 designed to deliver the gas needs for all filtration units, each of which have local pressure control. The off-gas conditioning vessel 2050 is designed as a vapor liquid separator and condensate surplus to that required for wetting is removed via line 2090.

The driving gas for filtration is taken from the reactor overheads line 1045, after removing most of the acetic acid present in rectification columns 1040, or after Absorber 1065 after further removal of volatile organic components (VOCs). Vapor at the point exiting the oxidation reactor is typically at 170 to 200°C and 8 to 13 bara, with vapor in line 1045 being typically at 160 to 165°C and 7 to 11bara. The vapor in line 1045 is composed of non-condensable gas which is high in nitrogen and low in oxygen along with still lower levels of carbon oxides, and is saturated with condensables. Condensable components are mainly water (>95%) with residual acetic acid and a number of other volatile organic components. The composition after Absorber 1065 is similar but still lower in volatile organic components, VOCs, which may deliver further contamination control benefits.

Driving gas for filtration is required at line 2080, where it should typically be 90 to 110°C and at 3-6 bara. Gas close to saturation with condensables is required, as this will limit evaporative cooling as the gas/vapor passes through the filter cake. Evaporative cooling is to be avoided as, in addition to energy loss, such cooling can lead to precipitation of soluble species in the filtrate and subsequent filter fouling. Thus, the gas/vapor should have a dew point of from 70 to 150°C, or from 80 to 135°C, or even from 90 to 110°C after conditioning. Thus, conditioning of the oxygen-depleted air to provide a conditioned oxygen-depleted air that is less than fully saturated (close to but safely and controllably above its dew point) at the temperature and pressure conditions prevalent before its use in filtration is desired.

The condensable species should be all or mostly water for effective solvent interchange from an acidic medium to a water based solvent.

The demisted gas exiting the off-gas conditioning vessel 2050 at point B is fully saturated, at typically 90 to 100°C, and at a pressure of 3 to 6 bara. However, if this gas becomes sub-saturated as the pressure falls downstream, highly corrosive species in the gas (hydrogen bromide) may be concentrated in a fog/mist as the gas dries out. This represents a significant metallurgical risk due to potential corrosion.

Use of exotic metallurgy downstream where a fog/mist could occur is possible but this is not considered practical or economic.

The alternative method of controlling this corrosion risk is to dry the gas at point B by controlled mixing with a small flow of additional hot and sub-saturated reactor off-gas removed via line 2060. This gas from line 2060, with flow temperature controlled by temperature control valve 2040, is then mixed with gas at point B in a relatively short length of highly corrosion resistant (exotic metallurgy) piping to effect a small temperature rise (typically 5 to 10°C). This temperature rise is chosen to be sufficient to reduce the gas humidity to a point where corrosive fog formation will not occur, yet retain sufficient moisture to minimize later evaporative cooling in the filtration step.

This conditioning of the reactor gas to ensure a temperature of typically 90 to 110°C. and just sufficiently sub-saturated to eliminate a fogging risk is now considered suitable for use as a filtration driving gas in the solvent interchange filter, or RPF. It can be advantageous to locally control the pressure within individual filters of the RPF.

Following combination of the CTA with aqueous medium to produce the terephthalic acid-containing slurry and subsequent heating to dissolve the CTA, the resulting solution may be fed directly to the hydrogenation step in a CTA purification reactor, or alternatively, may be treated prior to it being fed to the hydrogenation step.

Suitably the heterogeneous catalyst employed in the purification of the crude terephthalic acid product is a supported noble metal catalyst, for example platinum, rhodium and/or preferably palladium on an inert, for example carbon, support. The reduction is suitably carried out by passing the terephthalic acid solution comprising terephthalic acid and impurities, for example 4-carboxybenzaldehyde, through a flooded bed of catalyst at a temperature of 250 to 350°C. in the presence of hydrogen. The solution suitably comprises 20 to 50% by weight of terephthalic acid.

The terephthalic acid solution, after reduction, is suitably cooled in a crystallization process to a temperature in the range 100 to 220°C., typically 135 to 180°C., and a pressure of 3 to 10 bars, to produce solid purified terephthalic acid (PTA) product.

### Example

Oxygen-depleted air (hereinafter "gas") is supplied to a filter operating with a filtrate side pressure of typically 1.6barA and a cake pressure drop of typically 2bar. Gas flow to each filter is controlled for each filter therefore, allowing for control valve pressure drop and piping losses, a conditioned gas supply pressure 4barA is required.

Wet oxygen-depleted air is available for use as the gas to provide motive force for filtration. The preferred location to extract this gas is from the reactor overheads at pressure and approximately 135°C with a high water and low volatile organics (including brominated organics).

If this gas is let down to a pressure of 4-5 bara then used directly in the filtration step there will be significant evaporative cooling. In order to limit evaporative cooling in the filter area, the sub-saturated gas is mixed with condensate from the reactor overheads or recovered process water on pressure let-down to create an excess over-saturation. A suitable condensate is available from the reactor overheads at around 108°C. Process water is also available at around 112°C.

Mixing this gas and liquid with an excess on saturation of 30:1 and then pressure let-down to 4-5 bara results in suitable moisture content a at 94°C.

After gas liquid separation in a suitable separation device (for example a an impingement separator and proprietary mesh demister) the gas remains humid and contains trace quantities of hydrogen bromide, which is highly corrosive, particularly if some drying out of the gas is permitted to occur as pressure falls in downstream pipework and the filters. To avoid expensive materials of construction by upgrading from stainless steels to all piping and filters, for example to titanium, the gas is heated in a small section of piping or equipment constructed from the upgrade material. Suitable equipment would be a heat exchanger with resistant tubes, but can be more cost effectively performed by controlled mixing with a suitable hotter gas. Such a gas is already available from the reactor overheads where oxygen-depleted air is heated prior to catalytic treatment for VOC and CO removal at 100 to 150°C.

Mixing this saturated and heated gas in the ratio of approximately 5:1 results in a gas at approximately 100°C, which is considered to give adequate margin over saturation for corrosion control so long as an appropriate mixing length (typically no less than 10 pipe diameters), fabricated using the corrosion resistant material, is provided before downstream equipment metallurgy changes to more cost effective stainless steels. Gas flow of this hot gas is controlled on downstream temperature.

By using this conditioned off-gas as opposed to normal plant inert gas, evaporative cooling of the filter cake is reduced, yielding a filter cake and filtrate at typically around 90°C. With normal unconditioned plant inert gas the predicted cake discharge temperature is around 80°C, with the difference representing an energy loss from the process, due to a need for additional cake heating in the next process step and increased undesirable evaporation which is known to increase filter fouling due to additional precipitation or crystallization of soluble species in mother liquor.

### Additional benefits of using this conditioning method are:

Avoidance of the need to treat extra oxygen depleted air in a catalytic combustion unit (entailing extra capital cost through an increased size for the CCU and ancillary inert has treatment systems) along with inert gas required for other duties.

Reducing additional water ingress to the PTA process due to a need to re-wet dries inert gas.

Reducing terephthalic acid reheat required in the downstream purification step. For a typical state of the art plant with typical HP steam costs this can translate to several hundred thousand dollars per year saving in operating costs. This is in addition to the reduction in capital cost associated with avoiding the need for larger heating equipment.

### Industrial Applicability

The systems and methods disclosed herein are applicable to the chemical industry.

## Claims

1. An apparatus for the purification of crude terephthalic acid, comprising:
(a) a paraxylene oxidation reactor in which paraxylene is reacted with air in an acetic acid-based solvent containing catalyst to form a slurry containing crude terephthalic acid (CTA) crystals and an oxygen-depleted air off-gas;
(b) one or more crystallization vessels downstream of the reactor for flash cooling the slurry by pressure letdown;
(c) an off-gas conditioning system for removing and conditioning a portion of the oxygen-depleted air off-gas exiting the reactor;
(d) a rotary pressure filter for effecting solvent interchange in a first filtration step with counter-current washing utilizing the removed and conditioned oxygen-depleted air off-gas to pressurize an aqueous exchange solvent and to dissolve the CTA in the aqueous exchange solvent to produce a CTA-containing aqueous solution; and
(e) a CTA purification reactor in which the CTA-containing aqueous solution is contacted with hydrogen under reducing conditions and at elevated temperature and pressure to reduce chemically at least some impurities present in the CTA to form purified terephthalic acid (PTA).

2. The apparatus of claim 1, wherein the off-gas conditioning system comprises an inlet pipe connected downstream of the condensers and feeding an off-gas conditioning vessel, said off-gas conditioning vessel having an outlet pipe connected to an inlet of the rotary pressure filter.

3. The apparatus of claim 2, wherein the off-gas conditioning system further comprises a flow control valve and a pressure control valve along the inlet pipe to feed the off-gas to the off-gas conditioning vessel.

4. The apparatus of claim 3, wherein the off-gas conditioning system further comprises a temperature control valve connected between an off-gas heater which is downstream of the condensers and the outlet pipe from the off-gas conditioning vessel.

5. The apparatus of claim 4, wherein the flow control valve, the pressure control valve and the temperature control valve are controlled by a flow control loop, to condition and maintain a desired dew point of the off-gas in the off-gas conditioning vessel.

6. The apparatus of any preceding claim, further comprising a separation device communicating with the paraxylene oxidation reactor for separating a sub-saturated gas mixed with a condensate from an overhead of the paraxylene oxidation reactor to limit evaporative cooling in a filter area of the filter.

7. The apparatus of any one of claims 1 to 5, further comprising a separation device communicating with the paraxylene oxidation reactor for separating a sub-saturated gas mixed with process water to limit evaporative cooling in a filter area of the filter.

## Patentansprüche

1. Einrichtung zur Reinigung von roher Terephthalsäure, umfassend:
(a) einen Paraxylol-Oxidationsreaktor, in dem Paraxylol mit Luft in ein auf Essigsäure basierendes Lösungsmittel umgesetzt wird, das einen Katalysator beinhaltet, um einen Schlamm zu bilden, der Kristalle roher Terephthalsäure (CTA) und ein sauerstoffarmes Luftabgas beinhaltet;
(b) eines oder mehrere Kristallisierungsgefäße stromabwärts zum Reaktor zur Blitzkühlung des Schlamms durch Druckabbau;
(c) ein Abgasaufbereitungssystem zum Entfernen und Aufbereiten eines Teils des aus dem Reaktor austretenden sauerstoffarmen Luftabgases;
(d) einen Druckdrehfilter zum Bewirken von Lösungsmittelaustausch in einem ersten Filtrationsschritt mit Gegenstromwaschung unter Verwendung des entfernten und aufbereiteten sauerstoffarmen Luftabgases, um ein wässriges Austauschlösungsmittel unter Druck zu setzen und die CTA in dem Austauschlösungsmittel aufzulösen, um eine CTA-haltige wässrige Lösung zu erzeugen; und
(e) einen CTA-Reinigungsreaktor, in dem die CTA-haltige wässrige Lösung unter Reduktionsbedingungen und bei hoher Temperatur und Druck mit Wasserstoff in Kontakt gebracht wird, um mindestens einige der in den CTA vorhandenen Unreinheiten chemisch zu reduzieren, um gereinigte Terephthalsäure (PTA) zu bilden.

2. Einrichtung nach Anspruch 1, wobei das Abgasaufbereitungssystem eine Einlassleitung umfasst, die stromabwärts des Kondensators verbunden ist und ein Abgasaufbereitungsgefäß speist, wobei das Abgasaufbereitungsgefäß eine Auslassleitung aufweist, die mit einem Einlass des Druckdrehfilters verbunden ist.

3. Einrichtung nach Anspruch 2, wobei das Abgasaufbereitungssystem weiter ein Durchflussregelventil und ein Druckregelventil entlang der Einlassleitung umfasst, um das Abgas in das Abgasaufbereitungsgefäß einzuspeisen.

4. Einrichtung nach Anspruch 3, wobei das Abgasaufbereitungssystem weiter ein Temperaturregelventil umfasst, das zwischen einem Abgaserhitzer, der stromabwärts von den Kondensatoren liegt, und der Ausgabeleitung aus dem Abgasaufbereitungsgefäß verbunden ist.

5. Einrichtung nach Anspruch 4, wobei das Durchflussregelventil, das Druckregelventil und das Temperaturregelventil durch eine Durchflussregelschleife gesteuert werden, um einen gewünschten Taupunkt des Abgases in dem Abgasaufbereitungsgefäß zu bedingen und aufrechtzuerhalten.

6. Einrichtung nach einem vorstehenden Anspruch, weiter umfassend eine Trennvorrichtung, die mit dem Paraxylol-Oxidationsreaktor kommuniziert, um ein untersättigtes Gas, das mit einem Kondensat aus einem Overhead des Paraxylol-Oxidationsreaktors vermischt ist, zu trennen, um Verdampfungskühlung in einem Filterbereich des Filters zu begrenzen.

7. Einrichtung nach einem der Ansprüche 1 bis 5, weiter umfassend eine Trennvorrichtung, die mit dem Paraxylol-Oxidationsreaktor kommuniziert, um ein untersättigtes Gas, das mit Prozesswasser vermischt ist, zu trennen, um Verdampfungskühlung in einem Filterbereich des Filters zu begrenzen.

## Revendications

1. Appareil de purification d'acide téréphtalique, comprenant :
(a) un réacteur d'oxydation du paraxylène dans lequel le paraxylène est mis en réaction avec de l'air dans un solvant à base d'acide acétique contenant un catalyseur pour former une bouillie contenant des cristaux d'acide téréphtalique brut (CTA) et un gaz de dégagement d'air appauvri en oxygène ;
(b) un ou plusieurs récipients de cristallisation en aval du réacteur pour le refroidissement instantané de la bouillie par abaissement de pression ;
(c) un système de conditionnement de gaz de dégagement pour retirer et conditionner une partie du gaz de dégagement d'air appauvri en oxygène sortant du réacteur ;
(d) un filtre rotatif sous pression pour effectuer un échange de solvant dans une première étape de filtration avec lavage à contre-courant utilisant le gaz de dégagement d'air appauvri en oxygène retiré et conditionné pour mettre sous pression un solvant d'échange aqueux et pour dissoudre le CTA dans le solvant d'échange aqueux afin de produire une solution aqueuse contenant du CTA ; et
(e) un réacteur de purification de CTA dans lequel la solution aqueuse contenant le CTA est mise en contact avec de l'hydrogène dans des conditions de réduction et à une température et une pression élevées pour réduire chimiquement au moins certaines impuretés présentes dans le CTA afin de former de l'acide téréphtalique purifié (PTA).

2. Appareil selon la revendication 1, dans lequel le système de conditionnement de gaz de dégagement comprend un tuyau d'entrée connecté en aval des condenseurs et alimentant un récipient de conditionnement de gaz de dégagement, ledit récipient de conditionnement de gaz de dégagement présentant un tuyau de sortie connecté à une entrée du filtre rotatif sous pression.

3. Appareil selon la revendication 2, dans lequel le système de conditionnement de gaz de dégagement comprend en outre une vanne de régulation de débit et une vanne de régulation de pression le long du tuyau d'entrée pour amener le gaz de dégagement au récipient de conditionnement de gaz de dégagement.

4. Appareil selon la revendication 3, dans lequel le système de conditionnement de gaz de dégagement comprend en outre une vanne de régulation de température connectée entre un réchauffeur de gaz de dégagement qui est en aval des condenseurs et le tuyau de sortie du récipient de conditionnement de gaz de dégagement.

5. Appareil de la revendication 4, dans lequel la vanne de régulation du débit, la vanne de régulation de la pression et la vanne de régulation de la température sont commandées par une boucle de régulation du débit, pour conditionner et maintenir un point de rosée souhaité du gaz de dégagement dans le récipient de conditionnement de gaz de dégagement.

6. Appareil selon une quelconque revendication précédente, comprenant en outre un dispositif de séparation communiquant avec le réacteur d'oxydation du paraxylène pour séparer un gaz sous-saturé mélangé à un condensat provenant d'une tête du réacteur d'oxydation du paraxylène pour limiter le refroidissement par évaporation dans une zone de filtre du filtre.

7. Appareil selon l'une quelconque des revendications 1 à 5, comprenant en outre un dispositif de séparation communiquant avec le réacteur d'oxydation du paraxylène pour séparer un gaz sous-saturé mélangé à de l'eau de traitement afin de limiter le refroidissement par évaporation dans une zone du filtre.
